(19) Europäisches Patentamt
European Patent Office
Office européen des brevets

(11) Veröffentlichungsnummer: **0 079 310**
**B1**

(12) **EUROPÄISCHE PATENTSCHRIFT**

(45) Veröffentlichungstag der Patentschrift: **25.05.88**

(51) Int. Cl.$^4$: **C 07 D 211/46**

(21) Anmeldenummer: **82810467.9**

(22) Anmeldetag: **03.11.82**

# (54) Verfahren zur Herstellung von Polyalkylpiperidinölestergemischen.

(30) Priorität: **10.11.81 CH 7207/81**

(43) Veröffentlichungstag der Anmeldung:
**18.05.83 Patentblatt 83/20**

(45) Bekanntmachung des Hinweises auf die Patenterteilung:
**25.05.88 Patentblatt 88/21**

(84) Benannte Vertragsstaaten:
**CH DE FR GB IT LI NL**

(56) Entgegenhaltungen:
**EP-A-0 052 579**
**DE-A-2 257 997**

(73) Patentinhaber: **CIBA-GEIGY AG**
**Klybeckstrasse 141**
**CH-4002 Basel (CH)**

(72) Erfinder: **Meier, Anton**
**Matte 1**
**CH-4312 Magden (CH)**
Erfinder: **Troxler, Eduard, Dr.**
**St. Albanring 220**
**CH-4052 Basel (CH)**

**EP 0 079 310 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

In der nicht vorpublizierten veröffentlichten europäischen Patentanmeldung 52 579 wird die Herstellung eines auch gemäss vorliegender Anmeldung herstellbaren Estergemisches durch Umsetzung eines Diesters mit einem Piperidinol beschrieben, wobei gemäss Beispiel 1 Tetrabutylorthotitanat als Katalysator und Xylol als Lösungsmittel verwendet wird.

In der DE—A—2 257 997 wird die Herstellung von strukturell andersartigen Piperidinolestern beschrieben, wobei die Umesterung in der Schmelze und mit einem Alkalimetallamid als Katalysator stattfindet.

Aus US—A—4.021.432 sind Polyalkylpiperidinester als Lichtschutzmittel für Kunststoffe bekannt, wie das Bis-(1,2,2,6,6-penta-methyl-4-piperidyl)-sebacat. Für die praktische Anwendung haben sich solche bekannten Stabilisatoren nicht in allen Fällen als zufriedenstellend erwiesen. Insbesondere bei Einsatz fester Substanzen, gegebenenfalls auch in Form von Lösungen, ergeben sich in der Praxis Nachteile, die z.B. bei der Herstellung lichtstabiler Lacke, wie Autolacke, zu Problemen führen. So sind die bekannten Lichtschutzmittel für die Anwendung in lösungsmittelarmen Lacksystemen (high solids) nicht geeignet.

Die vorliegende Erfindung betrifft ein Verfahren zur Herstellung von Estergemischen von Polyalkylpiperidinderivaten der Formel (I) und (II)

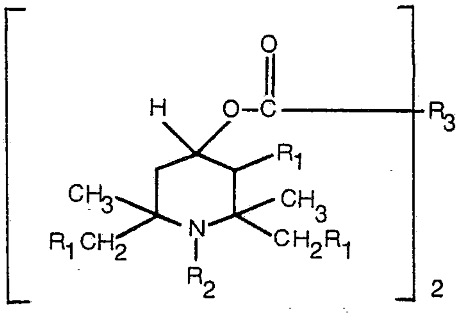

I

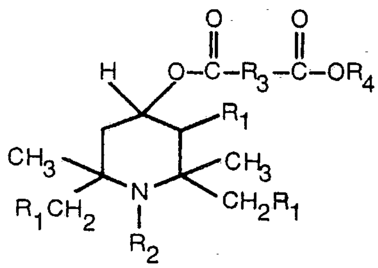

II

worin $R_1$ Wasserstoff oder Methyl ist, $R_2$ Wasserstoff, $C_{1-12}$Alkyl, Allyl, $C_{7-11}$Aralkyl, Cyanmethyl oder $C_{2-4}$Acyl ist, $R_3$ $C_{1-18}$Alkylen, $C_{2-18}$Oxaalkylen, $C_{2-18}$Thiaalkylen, $C_{2-18}$Azaalkylen oder $C_{2-8}$Alkenylen ist, $R_4$ $C_{1-4}$Alkyl bedeutet und das Verhältnis der beiden Ester zwischen 95 bis 70 Gew.-% (I) und 5 bis 30 Gew.-% (II) variiert, durch Umsetzung von ca. 2 Mol eines Piperidins der Formel (III)

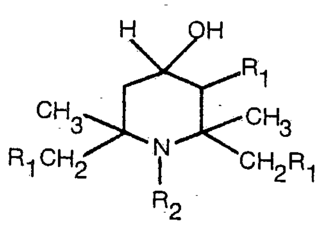

III

mit 0,9 bis 1,3 Mol eines Diesters der Formel (IV)

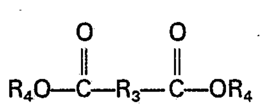

(IV)

worin $R_1$, $R_2$, $R_3$ und $R_4$ die angegebenen Bedeutungen haben, in Gegenwart eines Katalysators und bei einer Temperatur zwischen 100 und 145°C, dadurch gekennzeichnet, dass die Umsetzung in der Schmelze und mit einem Alkalimetallamid, bevorzugt Lithiumamid, als Katalysator stattfindet.

Nach der Umsetzung wird der gebildete Alkohol vorzugsweise im Vakuum, insbesondere bei einem Druck zwischen 0,6 bar und 10 mbar, ganz besonders bevorzugt bei 15 bis 25 mbar, abdestilliert. Die Aufarbeitung geschieht nach üblichen Methoden.

Bevorzugt werden ca. 2 Mol eines Piperidins der Formel (III) und 1,1 bis 1,25 Mol eines Diesters der Formel (IV) eingesetzt, um ein Estergemisch mit einem Verhältnis 90 bis 75 Gew.-% Ester (I) zu 10 bis 25 Gew.-% Ester (II) zu erhalten.

Die Umsetzung wird vorzugsweise bei einer Temperatur zwischen 125 und 140°C durchgeführt.

Die Ausgangsstoffe sind bekannt. Sollten einige von ihnen noch neu sein, so können sie in Analogie zu bekannten Verfahren hergestellt werden.

$R_2$ ist als $C_{1-12}$Alkyl insbesondere geradkettiges Alkyl mit insbesondere 1 bis 4 C-Atomen, wie Aethyl, n-Propyl, n-Butyl, und vor allem Methyl.

$R_2$ ist als $C_{7-11}$Aralkyl insbesondere Benzyl.

$R_2$ ist als $C_{2-4}$Acyl insbesondere Alkanoyl oder Alkenoyl, wie Propionyl, Acryloyl und vor allem Acetyl.

$R_3$ ist als $C_{1-18}$Alkylen verzweigtes oder insbesondere geradkettiges Alkylen, insbesondere solches mit 1 bis 10 C-Atomen, wie Methylen, Aethylen, Trimethylen, Tetramethylen, Hexamethylen, Decamethylen und insbesondere Octamethylen.

$R_3$ ist als $C_{2-18}$Oxaalkylen insbesondere geradkettiges Oxaalkylen mit insbesondere 2 bis 9 C-Atomen, wie 2-Oxa-trimethylen oder 3-Oxapentamethylen.

$R_3$ ist als $C_{2-18}$Thiaalkylen insbesondere geradkettiges Thiaalkylen mit insbesondere 2 bis 9 C-Atomen, wie 2-Thia-trimethylen oder 3-Thiapentamethylen.

$R_3$ ist als $C_{2-18}$Azaalkylen geradkettiges oder verzweigtes, insbesondere am Aza verzweigtes Azaalkylen mit insbesondere 2 bis 9 C-Atomen, wie 3-Aza-pentamethylen, 3-Aza-3-methyl-pentamethylen, 4-Aza-heptamethylen oder 4-Aza-4-methyl-heptamethylen.

$R_3$ ist als $C_{2-8}$Alkenylen insbesondere geradkettiges Alkenylen, wie Aethenylen oder 2-Buten-1,4-ylen.

$R_4$ ist als $C_{1-4}$Alkyl z.B. Aethyl, n-Propyl, i-Propyl, n-Butyl, i-Butyl oder insbesondere Methyl.

Das erfindungsgemässe Verfahren ist insbesondere für die Herstellung von Estergemischen der Formeln (I) und (II) geeignet, worin

$R_1$ Wasserstoff ist,

$R_2$ $C_{1-4}$ Alkyl oder Benzyl ist,

$R_3$ $C_{1-18}$ Alkylen ist, und

$R_4$ $C_{1-4}$Alkyl ist.

Das erfindungsgemässe Verfahren ist ganz besonders für die Herstellung von Estergemischen der Formeln (I) und (II) geeignet, worin

$R_1$ Wasserstoff ist,

$R_2$ Methyl oder Benzyl ist,

$R_3$ geradkettiges $C_{2-10}$Alkylen ist, und

$R_4$ $C_{1-4}$Alkyl ist.

Bevorzugt wird das erfindungsgemässe Verfahren zur Herstellung von Estergemischen der Formeln (I) und (II), worin

$R_1$ Wasserstoff ist,

$R_2$ Methyl ist,

$R_3$ Octamethylen ist, und

$R_4$ Methyl ist.

Es hat sich gezeigt, dass die nach dem erfindungsgemässen Verfahren erhaltenen Estergemische besonders gute Lichtschutzmittel für Kunststoffe sind, welche die oben bezüglich der in US—A—4.021.432 beschriebenen Polyalkylpiperidinester erwähnten Nachteile nicht oder aber in wesentlich geringerem Masse zeigen. Sie sind mit dem Substrat gut verträglich und können in diesem ohne Schwierigkeiten eingearbeitet werden. Sie verteilen sich schnell und homogen im Substrat, wobei letzteres wirksam und lang anhaltend gegen den schädlichen Einfluss von Licht geschützt wird. Durch das erfindungsgemässe Verfahren ist es möglich, die oben genannten Estergemische im gewünschten Verhältnis in quantitativer Ausbeute zu erhalten.

Die folgenden Beispiele erläutern die Erfindung.

## Beispiel 1

152,6 g' (0.663 Mol) Sebacinsäuredimethylester und 189,5 g (1,106 Mol) 1,2,2,6,6-Pentamethyl-4-hydroxypiperidin werden unter Stickstoff durch Erwärmen auf 95°C geschmolzen. Bei 95°C wird 1 g Lithiumamid zugegeben. Anschliessend wird auf 125° bis 130°C erwärmt wobei die Reaktion einsetzt indem Methanol abdestilliert. Nachdem ca. 30 ml Methanol abdestilliert sind, wird auf 100°C gekühlt und Vakuum von 0,6 bar angelegt. Nun wird unter Vakuum auf 140°C aufgeheizt wobei das Vakuum auf 20 bis 25 mbar sinkt. Nach 2 bis 3 Stunden Rühren bei 140°C ist die Reaktion beendet. Zur Aufarbeitung wird das Reaktionsgemisch auf 80°C gekühlt, mit 100 ml Benzin Fraktion (Siedepunkt 80 bis 110°C) versetzt, mit 50 ml 15 bis 20 Gew.-% wässriger Essigsäure extrahiert, anschliessend mit 50 ml Wasser nachextrahiert. Nach dem Abtrennen der wässrigen Phase wird die organische Phase azeotrop entwässert indem man das Gemisch auf 110°C aufheizt und über einen Abscheider das Wasser abtrennt. Nun wird über eine mit Filterhilfsmittel belegte Nutsche geklärt und das Benzin unter Vakuum bei 110°C abdestilliert. Ausbeute 283,2 g (quantitativ). Die erhaltene, schwach gelbliche, ölige Flüssigkeit ist ein Gemisch aus ca. 80% Sebacinsäure-bis-(1,2,2,6,6-pentamethyl-4-piperidinyl)-ester und ca. 20% Sebacinsäure-mono-(1,2,2,6,6-pentamethyl-4-piperidinyl)-monomethylester.

## Beispiel 2

Man verfährt gleich wie im Beispiel 1 setzt jedoch 139,9 g (0,609 Mol) Sebacinsäuredimethylester und 189,5 g (1,106 Mol) 1,2,2,6,6-Pentamethyl-4-hydroxypiperidin ein. Man erhält 271 g einer schwach gelblichen öligen Flüssigkeit bestehend aus ca 90% Sebacinsäure-bis-(1,2,2,6,6-pentamethyl-4-piperidinyl)-ester und ca. 10% Sebacinsäure-mono-(1,2,2,6,6-pentamethyl-4-piperidinyl)-monomethylester.

Beispiel 3

Man verfährt gleich wie im Beispiel 1 setzt jedoch 397,5 g (1,728 Mol) Sebacinsäuredimethylester und 473,8 g (2,765 Mol) 1,2,2,6,6-Pentamethyl-4-hydroxypiperidin ein. 2,5 g Lithiumamid als Katalysator sind notwendig. Die Extraktionen werden mit 130 ml einer 15 bis 20 Gew.-% wässrigen Essigsäure und mit 120 ml Wasser durchgeführt.

Man erhält 747,9 g einer schwach gelblichen öligen Flüssigkeit bestehend aus 75% Sebacinsäure-bis-(1,2,2,6,6-pentamethyl-4-piperidinyl)ester und ca 25% Sebacinsäure-mono-(1,2,2,6,6-pentamethyl-4-piperidinyl)-mono-methylester.

**Patentansprüche**

1. Verfahren zur Herstellung von Estergemischen von Polyalkylpiperidin-derivaten der Formeln (I) und (II)

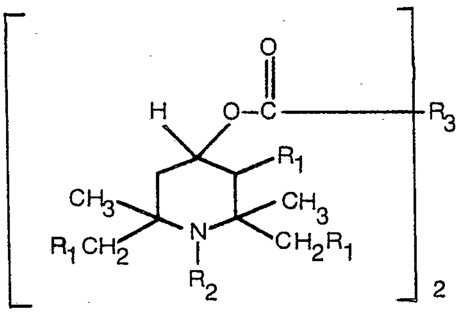

I

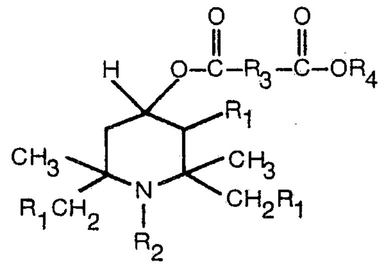

II

worin $R_1$ Wasserstoff oder Methyl ist,

$R_2$ Wasserstoff, $C_{1-12}$Alkyl, Allyl, $C_{7-11}$Aralkyl, Cyanmethyl oder $C_{2-4}$Acyl ist,

$R_3$ $C_{1-18}$-Alkylen, $C_{2-18}$Oxaalkylen, $C_{2-18}$Thiaalkylen, $C_{2-18}$Azaalkylen oder $C_{2-8}$Alkenylen ist, $R_4$ $C_{1-4}$Alkyl bedeutet und das Verhältnis der beiden Ester zwischen 95 bis 70 Gew.-% (I) und 5 bis 30 Gew.-% (II) variiert, durch Umsetzung von ca. 2 Mol eines Piperidins der Formel (III)

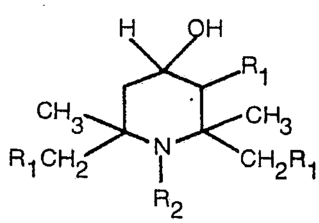

III

mit 0,9 bis 1,3 Mol eines Diesters der Formel (IV)

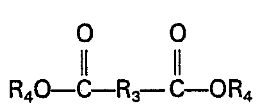

(IV)

worin $R_1$, $R_2$, $R_3$ und $R_4$ die angegebenen Bedeutungen haben, in Gegenwart eines Katalysators und bei einer Temperatur zwischen 100 und 145°C, dadurch gekennzeichnet, dass die Umsetzung in der Schmelze und mit einem Alkalimetallamid als Katalysator stattfindet.

2. Verfahren gemäss Anspruch 1, worin der bei der Umsetzung gebildete Alkohol im Vakuum abdestilliert wird.

3. Verfahren gemäss Anspruch 1, worin man bei einem Einsatz von ca. 2 Mol eines Piperidins der Formel (III) und 1,1 bis 1,25 Mol eines Diesters der Formel (IV) ein Estergemisch mit einem Verhältnis 90 bis 75 Gew.-% Ester (I) zu 10 bis 25 Gew.-% Ester (II) erhält.

4. Verfahren gemäss Anspruch 1, worin die Umsetzung bei einer Temperatur zwischen 125 und 140°C durchgeführt wird.

5. Verfahren gemäss Anspruch 1, worin Lithiumamid als Katalysator eingesetzt wird.

**Revendications**

1. Procédé pour préparer des mélanges d'esters de composés polyalkyl-pipéridiniques qui répondent aux formules I et II:

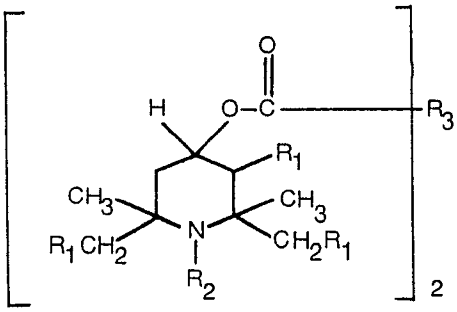

I

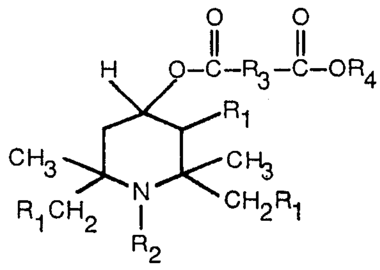

II

dans lesquelles:

$R_1$ représente l'hydrogène ou un méthyle,

$R_2$ représente l'hydrogène, un alkyle en $C_1$—$C_{12}$, un allyle, un aralkyle en $C_7$—$C_{11}$, un cyanométhyle ou un acyle en $C_2$—$C_4$,

$R_3$ représente un alkylène en $C_1$—$C_{18}$, un oxa-alkylène en $C_2$—$C_{18}$, un thia-alkylène en $C_2$—$C_{18}$, un aza-alkylène en $C_2$—$C_{18}$ ou un alcénylène en $C_2$—$C_{18}$ et

$R_4$ représente un alkyle en $C_1$—$C_4$, mélanges dans lesquels le rapport des deux esters est tel qu'il y ait de 95 à 70% en poids de (I) et de 5 à 30% en poids de (II), par réaction d'environ 2 mol d'une pipéridine de formule III:

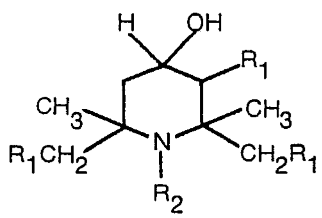

III

avec de 0,9 à 1,3 mol d'un diester de formule IV:

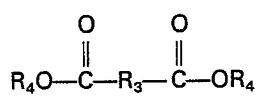

(IV)

formules dans lesquelles $R_1$, $R_2$, $R_3$ et $R_4$ ont les significations précédemment données, en présence d'un catalyseur et à une température comprise entre 100 et 145°C, procédé caractérisé en ce qu'on effectue la réaction à l'état fondu et en utilisant, comme catalyseur, un amidure de métal alcalin.

2. Procédé selon la revendication 1 dans lequel l'alcool formé au cours de la réaction est chassé par distillation sous pression réduite.

3. Procédé selon la revendication 1 dans lequel, en mettant en jeu environ 2 mol d'une pipéridine de formule III et de 1,1 à 1,25 mol d'un diester de formule IV, on obtient un mélange d'esters dans lequel le rapport de l'un à l'autre est tel qu'il y ait de 90 à 75% en poids de l'ester (I) pour 10 à 25% en poids de l'ester (II).

4. Procédé selon la revendication 1 dans lequel la réaction est effectuée à une température comprise entre 125 et 140°C.

5. Procédé selon la revendication 1 dans lequel on utilise l'amidure de lithium comme catalyseur.

**Claims**

1. A process for the preparation of a mixture of esters of polyalkylpiperidine derivatives, of the formulae (I) and (II)

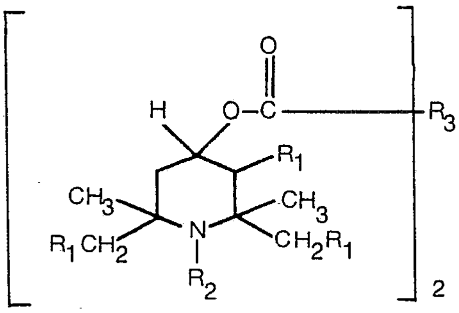

I

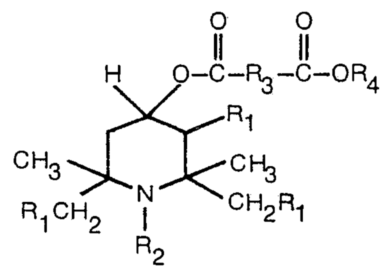

II

in which $R_1$ is hydrogen or methyl, $R_2$ is hydrogen, $C_{1-2}$alkyl, allyl, $C_{7-11}$aralkyl, cyanomethyl or $C_{2-4}$acyl, $R_3$ is $C_{1-18}$alkylene, $C_{2-18}$-oxaalkylene, $C_{2-18}$thiaalkylene, $C_{2-8}$azaalkylen or $C_{2-8}$alkenylen, $R_4$ is $C_{1-4}$alkyl and the proportions of the two esters vary between 95 to 70% by weight of (I) and 5 to 30% by weight of (II), by reacting about 2 mols of a piperidine of the formula (III)

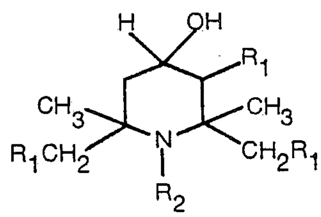

III

with 0.9 to 1.3 mols of a diester of the formula (IV)

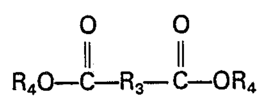

(IV)

in which $R_1$, $R_2$, $R_3$ and $R_4$ are as defined above, in the presence of a catalyst and at a temperature between 100 and 145°C, wherein the reaction takes place in the melt and with an alkali metal amide as the catalyst.

2. A process according to claim 1, wherein the alcohol formed during the reaction is distilled off in vacuo.

3. A process according to claim 1, wherein a mixture of esters with proportions of 90 to 75% by weight of ester (I) and 10 to 25% by weight of ester (II) is obtained when using about 2 mols of a piperidine of the formula (III) and 1.1 to 1.25 mols of a diester of the formula (IV).

4. A process according to claim 1, wherein the reaction is carried out at a temperature between 125 and 140°C.

5. A process according to claim 1, wherein lithium amide is used as the catalyst.